# EUROPEAN PATENT APPLICATION

(11) **EP 2 103 586 A1**
(43) Date of publication of application: **23.09.2009**
(21) Application number: 08250998.5
(22) Date of filing: 20.03.2008
(51) Int. Cl.: C07C 2/76

(54) **Process for converting methane into ethane in a membrane reactor**

(71) Applicant: BP OIL INTERNATIONAL LIMITED, Sunbury-on-Thames TW16 7BP (GB)
(72) Inventor: The designation of the inventor has not yet been filed
(74) Representative: De Kezel, Eric

(57) **Abstract**

The invention relates to a process for producing ethane comprising contacting methane with a metal catalyst selected from metal hydrides, organometallic compounds, organometallic hydrides and metal clusters, in a reaction zone and forming a reaction mixture comprising ethane and hydrogen, the reaction zone comprising a membrane reactor equipped with a selective hydrogen-permeable membrane. It also relates to the use of such a membrane reactor for activating the metal catalyst to reach its maximum activity during the initial reaction phase in the process for converting methane into ethane. The membrane can be selected from microporous inorganic membranes and dense inorganic membranes preferably chosen from metallic, ceramic or cermet (ceramic-metal) membranes, preferably from palladium and/or palladium alloy membranes and from proton-conducting ceramic membranes. One of the main advantages of the process relates to the methane conversion rate which very quickly reaches an extremely high maximum during the initial reaction phase of the conversion, so that the global methane conversion rate is very high, while maintaining a very high selectivity for ethane.

## Description

The invention relates to a process for converting methane into ethane carried out in a membrane reactor.

International Patent Application WO 03/104171 discloses a process for producing ethane comprising contacting methane with a metal catalyst selected from metal hydrides, metal organic compounds and mixtures thereof. The process involves methane non-oxidative coupling catalytic reaction leading to the formation of ethane and hydrogen. More particularly, it can comprise introducing methane into a reaction zone comprising the metal catalyst so as to form a gas mixture comprising ethane, hydrogen and unreacted methane, withdrawing the gas mixture from the reaction zone, separating the ethane in the withdrawn gas mixture from the unreacted methane and optionally the hydrogen, recovering the ethane and preferably returning the unreacted methane to the reaction zone. The process can be carried out in gas phase, in particular in a fluidized bed reactor and/or a reactor with a mechanically stirred bed, or in a stationary bed reactor or a circulating bed reactor, in which reactor the bed can be formed essentially with the metal catalyst.

Although in this process, the methane coupling catalytic reaction exhibits a very high selectivity for ethane with respect to carbon-containing products formed, it has been observed that the proportion of methane converted into ethane is relatively weak, essentially because the catalytic activity is very low in the initial phase of the reaction.

International Patent Application WO 02/053520 discloses a process for modifying aliphatic hydrocarbon chains, comprising reacting aliphatic hydrocarbons with themselves in the presence of a metal organic catalyst or its hydride. The reaction is carried out in the presence of hydrogen which preents the catalyst from rapidly deactivating. Hydrogen is preferably fed during the reaction.

It has been now found a process for converting methane into ethane with an extremely high initial catalyst activity and a very high global methane conversion, while maintaining the selectivity for ethane with respect to carbon-containing products formed to a very high level. Thus, in the process of the present invention, the methane conversion immediately reaches an extremely high level during the initial phase of the process which finally induces a very high global methane conversion.

The invention relates to a process for converting methane into ethane comprising contacting methane with a metal catalyst selected from metal hydrides, organometallic compounds, organometallic hydrides and metal clusters, in a reaction zone and forming a reaction mixture comprising ethane and hydrogen, characterised in that the reaction zone comprises a membrane reactor equipped with a selective hydrogen-permeable membrane.

The invention also relates to the use of a membrane reactor equipped with a selective hydrogen-permeable membrane for activating a metal catalyst to its maximum activity during the initial reaction phase in a process for converting methane into ethane comprising contacting methane with said metal catalyst selected from metal hydrides, organometallic compounds, organometallic hydrides and metal clusters, and forming a reaction mixture comprising ethane and hydrogen.

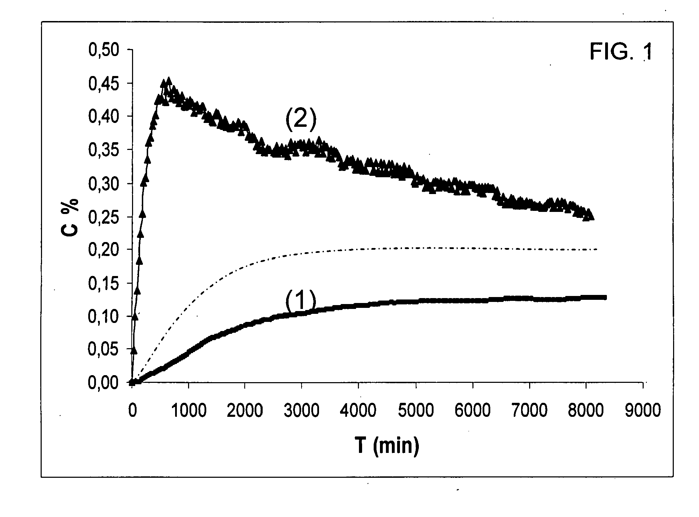

Figure 1 shows a diagram representing two kinetic curves (expressing the percentage of methane conversion (C %) versus time (T) in minutes) of the methane conversion performed under identical operating conditions, apart from being carried out in different reactors. Curve (1) shows the methane conversion as performed in Example 3 (comparative) in a standard reactor, and Curve (2) as performed in Example 4 according to the invention in a membrane reactor fitted with a selective hydrogen-permeable membrane.

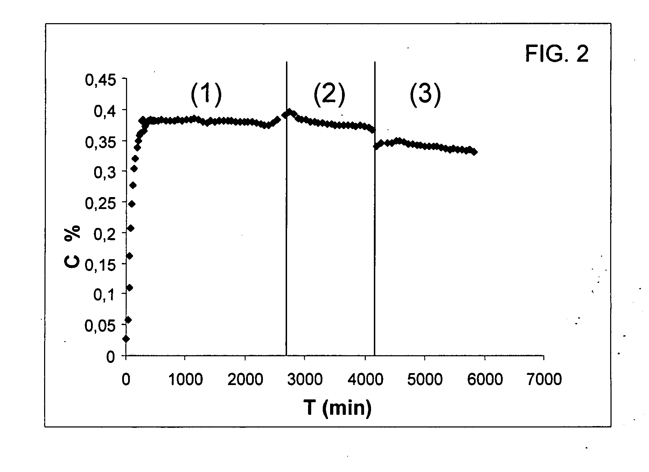

Figure 2 shows a diagram representing a kinetic curve (expressing the percentage of methane conversion (C %) versus time (T) in minutes) of the methane conversion performed in Example 6 according to the invention, under different process conditions.

The invention relates to a process for converting methane essentially into ethane, more particularly with a high conversion rate and a very high selectivity by weight for ethane with respect to carbon-containing products formed. The selectivity by weight for ethane is generally at least 90 %, preferably at least 95 %, particularly at least 98 % or even at least 99 %. The term "selectivity by weight for ethane" is generally understood to mean the part by weight of ethane produced per 100 parts by weight of carbon-containing products formed in the process. Advantageously, the process of the invention is able to produce ethane with a very high degree of purity, particularly without forming detectable amounts of carbon-containing products other than alkanes, especially without forming detectable amounts of alkenes (e.g. ethylene), alkynes (e.g. acetylene), aromatic compounds (e.g. benzene), carbon monoxide or carbon dioxide.

The present invention can be advantageously performed under conditions involving a non-oxidative catalytic coupling of methane. Preferably, it relates to a process carried out under operating conditions maintained substantially constant with time, preferably continuously. More particularly, it relates to a single-stage process for producing ethane.

The present invention relates to a process for producing ethane comprising contacting methane with a metal catalyst. The methane can be used alone or substantially alone, e.g. in the form of a mixture of methane with 1 % by mole or less of one or more other alkanes preferably selected from the (C₂₊) alkanes having 2 carbon atoms and more, particularly the C₂ to C₆ alkanes (hereafter referred as the (C₂₋₆) alkanes, as those present in natural gas. In this case, the methane can be obtained by fractionating natural gas, and separating and isolating the methane, in a preliminary stage comprising one or more methods suitable for fractionation of the natural gas, preferably chosen from any known methods or combinations of methods for fractionation of the natural gas, e.g. selected from the following six types of fractionation:
- fractionation by change of physical state, preferably of gaseous/liquid phase of the natural gas, in particular by distillation or by condensation (or liquefaction), more particularly by partial condensation (or partial liquefaction), in particular by means of a distillation/condensation column or tower,
- fractionation by molecular filtration, preferably by means of a semi-permeable and selective membrane,
- fractionation by adsorption, preferably by means of a molecular sieve or any other adsorbent,
- fractionation by absorption, in particular by means of an absorbing oil,
- fractionation by cryogenic expansion, preferably by means of an expansion turbine, and
- fractionation by compression, preferably by means of a gas compressor.

The methane can be also used in the process in the form of a mixture of methane, preferably in a major molar proportion, with one or more other alkanes preferably selected from the (C₂₊), particularly the (C₂₋₆) alkanes of the natural gas, especially in molar proportions similar or identical to the other alkanes existing in natural gas. In this case, the mixture of methane with one or more other alkanes may comprise a molar proportion of methane higher than 50 % and lower than 99 %, preferably higher than 60 % and lower than 99 %, particularly higher than 75 % and lower than 99 %. More particularly, the methane used in the process can be used in the form of natural gas, or can be substituted by natural gas.

The methane is preferably used in the process alone or substantially alone, as previously described.

The methane used in the process or the natural gas which can substitute the methane or from which the methane can be derived and used in the process, may comprise one or more poisons capable of particularly deactivating the metal catalyst, and optionally one or more impurities such as nitrogen (N₂) or nitrogen containing compounds. Thus, the process advantageously may comprise a prior purification stage comprising removing from the methane or the natural gas, one or more poisons and/or impurities, preferably selected from water, carbon dioxide, sulphur compounds especially chosen from hydrogen sulphide, carbonyl sulphide and mercaptans, and nitrogen (N₂) or nitrogen containing compounds. Any known methods can be used for removing these poisons and/or impurities.

The process comprises contacting methane with a metal catalyst which is selected from metal hydrides, organometallic compounds, organometallic hydrides and metal clusters, preferably supported on a solid support.

The metal catalyst can be chosen from metal clusters, preferably from supported metal clusters, comprising one or more metals of Groups 4, 5 and/or 6 of the Periodic Table of the Elements, preferably tantalum. The Periodic Table of the Elements (here and hereafter) is that proposed by IUPAC in 1991 and for example published by CRC Press, Inc. (USA) in "CRC Handbook of Chemistry and Physics" 76^{th} Edition (1995-1996), by David R. Lide. The supported metal clusters generally comprise a solid support and a metal cluster, and preferably comprise bonding between the metals of the metal cluster, and also bonding between the metal cluster and the solid support. Thus, the metal atom of the metal cluster can be bonded to at least one other metal atom of the metal cluster, preferably to six or more metal atoms of the metal cluster. The metal atom of the metal cluster can be bonded in addition to at least one hydrocarbon radical and/or to at least one hydrogen atom. The solid support can be chosen from a metal oxide, a refractory oxide, a molecular sieve, a zeolite, a metal phosphate and a material comprising a metal and oxygen, preferably silica or alumina. More particularly, the supported metal clusters can comprise tantalum (as a metal cluster) and silica (as a solid support), and preferably comprise bonding between tantalum and tantalum, and also bonding between tantalum and silica, in particular bonding between tantalum and oxygen on the silica surface. The metal catalyst can be chosen from the supported metal clusters described in International Patent Application WO 2006/060692, and prepared according to any methods described in said Application.

The metal catalyst is preferably selected from metal hydrides, organometallic compounds and organometallic hydrides, particularly supported on and more specifically grafted onto a solid support. It can comprise one or more metals chosen from lanthanides, actinides and metals of Groups 2 to 12, preferably from transition metals of Groups 3 to 12, more particularly Groups 3 to 10 of the Periodic Table of the Elements. In particular, the metal of the catalyst can be chosen from scandium, yttrium, lanthanum, titanium, zirconium, hafnium, vanadium, niobium, tantalum, chromium, molybdenum, tungsten, rhenium, ruthenium, osmium, nickel, iridium, palladium, platinum, cerium and neodymium. It can be preferably chosen from yttrium, titanium, zirconium, hafnium, vanadium, niobium, tantalum, chromium, molybdenum, tungsten, rhenium, ruthenium and platinum, and more especially from niobium, tantalum, molybdenum, tungsten and rhenium.

The metal catalyst chosen preferably from organometallic compounds and organometallic hydrides, can comprise one or more hydrocarbon radicals (R), either saturated or unsaturated, linear or branched, having preferably from 1 to 20, particularly from 1 to 14 carbon atoms. The hydrocarbon radicals (R) can be chosen from alkyl radicals, particularly either saturated or unsaturated, linear or branched, aliphatic or alicyclic, for example alkyl, alkylidene or alkylidyne radicals, preferably from C₁ to C₁₀, from aryl radicals preferably from C₆ to C₁₂, and from aralkyl, aralkylidene or aralkylidyne radicals preferably from C₇ to C₁₄. The metal atom of the catalyst can be preferably bonded to at least one hydrogen atom and/or to at least one hydrocarbon radical (R), in particular by a single, double or triple carbon-metal bond.

The metal catalyst preferably selected from metal hydrides, organometallic compounds and organometallic hydrides, can furthermore comprise one or more ligands, such as "ancillary" ligands preferably comprising at least one oxygen atom and/or at least one nitrogen atom. The ligands can be chosen from oxo, alkoxo, aryloxo, aralkyloxo, nitrido, imido and amido ligands. Generally, by oxo, alkoxo, aryloxo, aralkyloxo, nitrido, imido and amido ligands are respectively meant:
- a divalent oxo radical of general formula: = O
- a monovalent alkoxo, aryloxo or aralkyloxo radical of general formula: - OR'
- a trivalent nitrido radical of general formula: ≡ N
- a divalent imido radical of general formula: = NR", and
- a monovalent amido radical of general formula: - NR¹R², general formulae in which O represents an oxygen atom, N represents a nitrogen atom, R' represents an hydrogen atom or a monovalent hydrocarbon radical selected respectively from alkyl, aryl and aralkyl radicals, R" represents an hydrogen atom or a monovalent hydrocarbon radical, and R¹ and R² being identical or different represent an hydrogen atom or a monovalent hydrocarbon radical. More particularly, R', R", R¹ and R² can be a monovalent hydrocarbon radical either saturated or unsaturated, linear or branched, comprising from 1 to 20, preferably from 1 to 14 carbon atoms. They can be chosen from alkyl radicals preferably from C₁ to C₁₀, aralkyl radicals preferably from C₇ to C₁₄, and aryl radicals preferably from C₆ to C₁₂.

When the metal catalyst preferably from metal hydrides, organometallic compounds and organometallic hydrides comprises at least one of the above-mentioned ligands, the metal of the catalyst is not only bonded to at least one hydrogen atom and/or to at least one hydrocarbon radical (R), but also to at least one of these ligands, e.g. to the O atom of the oxo radical by a double bond (= O), or to the O atom of the alkoxo, aryloxo or aralkyloxo radical by a single bond (- OR'), or to the N atom of the nitrido radical by a triple bond (≡N), or to the N atom of the imido radical by a double bond (= NR"), or to the N atom of the amido radical by a single bond (- NR¹R²). Generally, the presence of the oxo, alkoxo, aralkyloxo, aryloxo, nitrido, imido and/or amido ligands in the metal catalyst favourably influences the behaviour of the metal catalyst in the methane coupling reaction.

In addition, the metal catalyst selected from metal hydrides, organometallic compounds and organometallic hydrides is preferably supported on, especially grafted onto a solid support. The solid support can be chosen from metal oxides, metal sulphides, metal carbides, metal nitrides, metal salts, refractory oxides, refractory sulphides, refractory carbides, refractory nitrides and refractory salts, and from carbon, mesoporous materials, organic or inorganic hybrid materials, metals and molecular sieves. The solid support can be chosen from silicas, aluminas, silica-aluminas and aluminium silicates, either simple or modified by other metals, and from zeolites, clays, titanium oxide, cerium oxide, magnesium oxide, niobium oxide, tantalum oxide and zirconium oxide. The solid support can be preferably chosen from silicas, aluminas, silica-aluminas and aluminium silicates, either simple or modified by other metals. It can have a specific surface area (B.E.T.) measured according to the standard ISO 9277 (1995) and chosen in a range of from 0.1 to 5000 m²/g, preferably from 1 to 3000 m²/g, more particularly from 1 to 1000 m²/g.

It is preferred to use a metal catalyst selected from metal hydrides, organometallic compounds and organometallic hydrides, and grafted onto a solid support preferably selected from the above-mentioned solid supports. In such a metal catalyst grafted onto a solid support, the metal atom can be bonded to one or more hydrogen atoms and/or to one or more hydrocarbon radicals (R), and also to one or more of the atoms of the support, preferably to one or more atoms of oxygen, sulphur, carbon or nitrogen of the support, particularly when the support is chosen from metal oxides, metal sulphides, metal carbides, metal nitrides, metal salts, refractory oxides, refractory sulphides, refractory carbides, refractory nitrides and refractory salts. The metal catalyst is preferably selected from metal hydrides and organometallic hydrides grafted onto a solid support, for example selected from niobium hydrides or organometallic niobium hydrides grafted onto silica, tantalum hydrides or organometallic tantalum hydrides grafted onto silica, molybdenum hydrides or organometallic molybdenum hydrides grafted onto silica, tungsten hydrides or organometallic tungsten hydrides grafted onto alumina, and rhenium hydrides or organometallic rhenium hydrides grafted onto silica.

The metal catalyst selected from metal hydrides, organometallic compounds and organometallic hydrides can be chosen from the metal catalysts described in International Patent Applications WO 98/02244, WO 03/061823 or WO 2004/089541, and prepared according to any of the methods described in said Applications.

In the process, the methane in contact with the metal catalyst generally reacts with itself and produce ethane and hydrogen by a methane non-oxidative coupling reaction, in particular described according to the following reverse equation (1):

2 CH₄ → C₂H₆ + H₂ (1)

In the process, the contacting generally leads to the formation of a reaction mixture comprising ethane and hydrogen and also generally unreacted methane. Under the operating conditions of the contacting, the reaction mixture is generally obtained in gaseous form. The contacting can be performed under conditions especially suited to promoting the development of the reaction towards an optimum production of ethane. Thus, it can be performed at a temperature chosen in a range of from 150 to 700°C, preferably from 200 to 650°C, particularly from 250 to 600°C, especially from 300 to 600°C. It can also be performed under an absolute total pressure, P', chosen in a range of from 0.1 to 50 MPa, preferably from 0.1 to 30 MPa, particularly from 0.1 to 20 MPa. It can also be performed in the presence of at least one inert agent, either liquid or gaseous, particularly an inert gas preferably selected from nitrogen, helium and argon.

If the methane is used in the process in the form of a mixture of methane with one or more other alkanes, the process may involve secondary reactions simultaneously developing with the methane non-oxidative coupling reaction. For instance, the process may involve secondary reactions chosen from self- and cross-metathesis reactions between one or more other alkanes, and also methane-olysis reactions between the methane and the other alkanes, these reactions being essentially catalysed with the metal catalyst used for the present process.

The contacting can be performed in various ways in a reaction zone, either intermittently (or discontinuously) or preferably continuously. For example, it is possible to add the methane to the metal catalyst, or to add the metal catalyst to the methane, or else to add the methane and the metal catalyst simultaneously into the reaction zone. It is preferred to add the methane to the metal catalyst in the reaction zone continuously.

According to the present invention, the reaction zone comprises a membrane reactor equipped with a selective hydrogen-permeable membrane (or a selective hydrogen-permeating membrane). Generally, a selective hydrogen-permeable membrane is also known as a hydrogen-permselective membrane, the term "permselective" generally referring to a membrane exhibiting selective permeation for hydrogen in a mixture comprising other gases, or also known as a hydrogen-selective, hydrogen-permeable membrane. A selective hydrogen-permeable membrane is generally selected from membranes having a high selective hydrogen permeance. More particularly, a selective hydrogen-permeable membrane used in the present invention is capable of selectively separating by permeation hydrogen from hydrocarbon mixtures, particularly from mixtures comprising ethane and hydrogen, especially from mixtures comprising ethane, hydrogen and methane.

The selective hydrogen-permeable membrane is preferably selected from (i) microporous inorganic membranes and (ii) dense inorganic membranes. These membranes are generally supported on a porous inorganic support preferably selected from porous metallic supports, porous (stainless) steel supports, porous ceramic supports, porous glass supports and porous refractory oxide supports.

According to the IUPAC definition, microporous inorganic membranes are generally referred to those porous membranes with a pore diameter smaller than 2 nm. Microporous inorganic membranes can be prepared as a thin film supported on a porous inorganic support which generally provides mechanical strength, but offers minimum mass-transfer resistance. More particularly, the selective hydrogen-permeable membrane used in the present invention can be selected from selective hydrogen-permeable microporous inorganic membranes.

According to the IUPAC definition, dense inorganic membranes are generally referred to those membranes made of a polycrystalline ceramic or metal which generally allows at least one specific gas to selectively permeate through its crystal lattice. Dense inorganic (essentially metallic) membranes can be prepared as a thin film on porous inorganic support, particularly on porous metal support or porous metal alloy support. More particularly, the selective hydrogen-permeable membrane used in the present invention can be selected from selective hydrogen-permeable dense inorganic membranes.

The selective hydrogen-permeable membrane can be a microporous inorganic membranes preferably supported on a porous inorganic support. The porous inorganic support can be selected from porous metallic supports (e.g. a porous "Inconel®" alloy support, or a porous "Hastelloy®" alloy support), porous (stainless) steel supports, porous ceramic supports, porous glass supports (e.g. a porous "Vycor"® support) and porous refractory oxide supports (e.g. a γ-alumina (a gamma-alumina) support). More particularly, a microporous inorganic membrane can be selected from microporous silica membranes, microporous alumina membranes, microporous zirconia membranes, microporous aluminium-doped silica membranes, microporous binary alumina-silica membranes, microporous binary zirconia-silica membranes and microporous binary zirconia-alumina membranes, which generally are supported on a porous inorganic support preferably selected from porous metallic supports, porous (stainless) steel supports, porous ceramic supports, porous glass supports and porous refractory oxide supports. For instance, a supported microporous silica membrane can be prepared by dip-coating an acid catalysed polymeric silica sol on a (meso)porous sol-gel derived γ-alumina support. Amongst the microporous silica membranes, it is preferred to use a microporous silica hollow fiber membrane which generally exhibits excellent hydrogen separation and selectivity properties particularly with respect to mixtures comprising hydrogen, methane and ethane. Such a microporous silica hollow fiber membrane can be prepared by phase separation method.

The selective hydrogen-permeable membrane can be also a microporous inorganic membrane selected from zeolite membranes, particularly from polycrystalline zeolite membranes preferably supported on a porous inorganic support which can be selected from porous metallic supports (e.g. a porous "Inconel®" alloy support, or a porous "Hastelloy®" alloy support), porous (stainless) steel supports, porous ceramic supports, porous glass supports (e.g. a porous "Vycor"® support) and porous refractory oxide supports (e.g. a γ-alumina support). For instance, a supported zeolite membrane can be prepared by growing a zeolite film on a porous inorganic support.

The selective hydrogen-permeable membrane can be preferably selected from dense inorganic membranes, particularly from dense metallic membranes, dense ceramic membranes and dense cermet (ceramic-metal) membranes, preferably supported on a porous inorganic support. The porous inorganic support can be selected from porous metallic supports (e.g. a porous "Inconel®" alloy support, or a porous "Hastelloy®" alloy support), porous (stainless) steel supports, porous ceramic supports, porous glass supports (e.g. a porous "Vycor"®) and porous refractory oxide supports (e.g. a γ-alumina support).

A dense metallic membrane can be preferably supported on a porous inorganic support, particularly with an asymmetric multilayer structure. A dense metallic membrane generally comprises one or more metals selected from Groups 4 to 11, preferably Groups 4 to 6 and Groups 8 to 11, e.g. from titanium, zirconium, vanadium, niobium, tantalum, molybdenum, iron, nickel, palladium, platinum and copper, especially from niobium, zirconium, palladium, vanadium, tantalum and titanium, or from Groups 8 to 11, e.g. from iron, nickel, palladium, platinum and copper, more particularly from Group 10 of the Periodic Table of the Elements, e.g. from nickel, palladium and platinum, especially palladium. A dense metallic membrane can be of many types, such as selected from (i) pure metals: palladium, vanadium, tantalum, niobium and titanium; (ii) binary alloys of palladium: palladium-copper, palladium-silver, palladium-yttrium, palladium-ruthenium and also palladium alloyed with nickel, gold, cerium and iron; (iii) complex alloys: palladium alloyed with 3 to 5 other metals; (iv) amorphous alloys: typically metals of Groups 4 to 6 of the Periodic Table of the Elements; and (v) coated metals: palladium over tantalum, vanadium, niobium and titanium. A dense metallic membrane is generally supported on a porous inorganic support which can be selected from a metallic support, a porous (stainless) steel support or a porous ceramic support (e.g. a porous ceramic α-alumina (alpha-alumina) support). The porous inorganic support can be particularly selected from (i) (stainless) steel, (ii) an alloy comprising chromium and nickel, (iii) a nickel-based alloy, and (iv) an alloy comprising chromium, nickel and molybdenum, e.g. an alloy comprising chromium, nickel and optionally molybdenum selected from porous "Hastelloy"® alloys (e.g. "Hastelloy C-22®") and porous "Inconel"® alloys (e.g. "Inconel alloy 625®"). A dense metallic membrane can be prepared by any known method, e.g. selected from electroless-plating, chemical vapour deposition and physical sputtering.

More particularly, a dense metallic membrane can be a palladium and/or palladium alloy membrane, preferably a palladium and/or palladium/silver alloy membrane, in particular supported on a porous metallic or ceramic support. A palladium alloy membrane .may comprise palladium and one or more other metals selected from cerium, yttrium, ruthenium, osmium, nickel, platinum, copper, silver and gold, preferably from yttrium, ruthenium, copper and silver, more particularly from silver. The other metal(s), preferably silver, yttrium and/or copper, may be present in the palladium alloy membrane in a range of from 10 to 70 %, preferably from 10 to 50 % by weight. The palladium alloy can be selected for instance from alloys comprising 77 wt % of palladium and 23 wt % of silver, or 75 wt % of palladium and 25 wt % of silver, or 90 wt % of palladium and 10 wt % of yttrium, or 60 wt % of palladium and 40 wt % of copper. A palladium and/or palladium/silver alloy membrane supported on a porous metallic or ceramic support is preferably used in the present invention. More particularly, it can be preferably used a palladium-coated membrane supported on a porous metallic support, a palladium/silver-coated membrane supported on a porous metallic support, a palladium- and palladium/silver-coated membrane supported on a porous metallic support, a palladium-coated membrane supported on a porous ceramic support, a palladium-silver coated ceramic membrane supported on a porous ceramic support, or a palladium- and palladium/silver-coated membrane supported on a porous ceramic support.

A palladium and/or palladium alloy membrane can be selected from the membranes described in American Patents US 4,857,080, US 5,215,729, US 5,931,987 and US 6,183,543 B1, and International Patent Application WO 2006/034103 A1, and prepared according to any of the methods described in said Patents and Application.

A dense metallic membrane can be prepared by coating a thin metal film on a porous inorganic support, e.g. a porous metal, (stainless) steel or ceramic support. A dense metallic membrane (or more specifically a supported dense metallic membrane) generally comprises a dense metallic film having a thickness of from 0.001 to 10 µm, preferably from 0.01 to 3 µm, particularly from 0.05 to 2 µm, generally deposited on a porous inorganic support which can have a thickness of from 50 to 5000 µm, preferably from 100 to 3000 µm. In general, the membrane thickness can be any useful thickness which will provide the desired hydrogen flux through the membrane at the desired reaction conditions and that will provide the sufficient mechanical strength to withstand the reaction conditions. A dense metallic membrane, such as a palladium or palladium alloy membrane, preferably supported on a porous inorganic support, is particularly useful in the present process generally carried out at high temperatures, e.g. from 150 to 700°C, preferably from 200 to 650°C, particularly from 250 to 600°C, especially from 300 to 600°C.

The selective hydrogen-permeable membrane can be also preferably selected from dense ceramic membranes, also generally called proton-conducting ceramic membranes, or more specifically perovskite type proton-conducting ceramic membranes, which may also be supported on a porous inorganic support particularly selected from porous metallic supports, porous (stainless) steel supports, porous ceramic supports, porous glass supports and porous refractory oxide supports. A dense ceramic membrane or more particularly a proton-conducting ceramic membrane, such as a perovskite type proton-conducting ceramic membrane, is generally based on the following general formula (2):

A B₍₁₋ₓ₎ B'ₓ O₃ (2)

general formula wherein A represents a calcium, strontium or barium atom, preferably a strontium or barium atom, B represents an atom of cerium, B' represents a titanium, vanadium, chromium, manganese, iron, copper, yttrium, praseodymium, terbium, thulium or ytterbium atom, preferably a thulium atom, and x is a number of from 0 to 0.2, preferably from 0 to 0.1. A dense ceramic membrane or more specifically a proton-conducting ceramic membrane, such as a perovskite type proton-conducting ceramic membrane, may be preferably based on one of the following general formulae (3) to (8):

Sr Ce O₃ (3)

Sr Ce₍₁₋ₓ₎ Ybₓ O₃ (4)

Sr Ce₍₁₋ₓ₎ Yₓ O₃ (5)

Ba Ce₍₁₋ₓ₎ Yₓ O₃ (6)

Sr Ce₍₁₋ₓ₎ Tbₓ O₃ (7)

Sr Ce₍₁₋ₓ₎ Tmₓ O₃ (8)

general formulae wherein Sr represents a strontium atom, Ce represents a cerium atom, O represents an oxygen atom, Yb represents an ytterbium atom, Y represents an yttrium atom, Ba represents a barium atom, Tb represents a terbium atom, Tm represents a thulium atom, and x is a number of from 0.02 to 0.08, preferably equal to 0.05. In such a dense ceramic membrane or more specifically a proton-conducting ceramic membrane, such as a perovskite type proton-conducting ceramic membrane, the membrane can have a thickness of from 0.5 to 5 mm, preferably from 1 to 3 mm, particularly from 1 to 2 mm. The dense ceramic membranes or more specifically the proton-conducting ceramic membranes, such as perovskite type proton-conducting ceramic membranes, are useful at very high temperatures, particularly in the present process generally carried out at a temperature of from 150 to 700°C, preferably from 200 to 650°C, particularly from 250 to 600°C, especially from 300°C to 600°C.

The selective hydrogen-permeable membrane can be also selected from dense cermets (ceramic-metal) membranes wherein dense cermets are generally prepared by mixing and sintering together powders of hydrogen-permeable metals and alloys with powders of ceramics. In a dense cermet membrane, the ceramic is generally selected from the ceramics present in the above-mentioned hydrogen-permeable ceramic membranes, preferably the proton-conducting ceramic membranes, more particularly the perovskite type proton-conducting ceramic membranes. In a dense cermet membrane, the metal(s) is(are) generally selected from the metals present in the above-mentioned hydrogen permeable metallic membranes.

Dense metallic, ceramic or cermet (ceramic-metal) membranes are particularly described in American Patents US 3,350,846, US 5,217,506, US 5,415,891, US 5,652,020, US 5,738,708, US 6,037,514, US 6,066,307, US 6,527,833, US 6,899,744 B2 and US 7,001,446 B2, American Patent Applications US 2003/0222015 A1 and US 2008/0000350 A1, and International Patent Applications WO 2006/017022 A2 and WO 2006/034103, and can be prepared according to any methods described in said Patents and Applications.

The contacting of the methane with the metal catalyst is carried out in a reaction zone comprising a membrane reactor equipped with the selective hydrogen-permeable membrane which can be in the form of a planar or tubular structure and generally comprises a reaction side and a permeate side. The reaction side of the membrane generally faces the metal catalyst and the reaction mixture present in the interior of the membrane reactor wherein the contacting is performed generally under the above-mentioned absolute total pressure (P'). The permeate side of the membrane generally faces the exterior of the membrane reactor which is generally submitted to an external absolute pressure (P") lower than P', so that it results a pressure difference (ΔP = P' - P") through the membrane. The pressure difference (ΔP) can be chosen in a range of from 0.05 to 5 MPa, preferably from 0.1 to 2 MPa, particularly from 0.2 to 1 MPa. The pressure difference (ΔP) generally acts as driving force which helps the hydrogen of the reaction mixture to selectively pass by permeation through the membrane from the reaction side to the permeate side of the membrane. The permeate side of the membrane generally delivers the hydrogen thus separated from the reaction mixture in the form of a permeate gas. Preferably, the permeate side of the membrane can be subjected to a sweep gas flow, in particular under the external absolute pressure (P"), so as to entrain the permeate gas, i.e. the hydrogen, externally to the membrane and to form a permeate gas mixture comprising the hydrogen and the sweep gas. In this case, the membrane reactor is preferably selected from sweep-flow membrane reactors. Generally, the sweep gas can be an inert gas preferably selected from nitrogen, argon and helium. The hydrogen can be separated and isolated from the permeate gas mixture by any known fractionation methods particularly capable of separating the hydrogen from the sweep gas. The hydrogen thus separated can be recovered and used for various applications. Thus, the hydrogen thus recovered can be used as an agent for activation or regeneration of the metal catalyst used in the contacting, particularly by contacting the metal catalyst in a stage distinct and separate from the process. The hydrogen can be also used in other applications distinct or not from the present process, for example as a fuel for producing thermal and/or electrical energies, in particular as a fuel in hydrogen fuel cells for producing electrical energy, such energies being preferably employed for running the present process, e.g. in purification or fractionation stages, or as a fuel for automobile, or as a reagent in a chemical, petrochemical or refinery plant.

The membrane reactor can be a gas phase membrane reactor containing the metal catalyst preferably in the form of solid particles or a bed, and equipped with the selective hydrogen-permeable membrane. The gas phase membrane reactor is preferably selected from static membrane reactors, recycling membrane reactors and dynamic membrane reactors. For instance, the contacting can be performed in a static membrane reactor generally containing fixed quantities of the metal catalyst and of the methane. The contacting can be also performed in a recycling membrane reactor preferably containing the metal catalyst and wherein at least one component of the reaction mixture, for example the ethane and the unreacted methane optionally with hydrogen, can be recycled preferably continuously. The contacting can be also performed in a dynamic membrane reactor containing the metal catalyst preferably in the form of solid particles or a bed, through which the methane can pass or circulate preferably continuously.

The membrane reactor can be a gas phase membrane reactor containing the metal catalyst preferably in the form of solid particles or a bed, equipped with the selective hydrogen-permeable membrane. The gas phase membrane reactor can be selected from tubular (or multi-tubular) reactors, distillation/condensation column or tower reactors, fluidised bed reactors, mechanically agitated bed reactors, fluidised and mechanically agitated bed reactors, fixed bed reactors, moving bed reactors and circulating bed reactors. The metal catalyst can be arranged inside or outside the tubes of a tubular (or multi-tubular) reactor. It can be also arranged inside of a distillation/condensation column or tower reactor functioning as both a catalyst and a distillation/condensation packing, i.e. a packing for a distillation/condensation column or tower having both a distillation/condensation function and a catalytic function as described in American Patent US 4,242,530. For example, it can be used in the form of rings, saddles, balls, granulates, irregular shapes, sheets, tubes, spirals or packed in bags. The metal catalyst can also form the bed of a fluidised and/or mechanically agitated bed, fixed bed, moving bed or circulating bed of the above-mentioned bed reactors. The methane can be introduced in the form of a stream preferably continuously into said reactors, and particularly can pass or circulate into or along the tubes, or through the bed or the distillation/condensation packing of said reactors.

The main advantage of the process of the invention essentially relates to a methane conversion process performed with an extremely high initial catalyst activity, while maintaining a very high level of selectivity by weight for ethane with respect to carbon-containing products formed. As shown by Curve (2) of Figure 1 (see Example 4), in the presence of the selective hydrogen-permeable membrane, the methane conversion during the initial reaction phase very quickly reached a maximum at an extremely high level, which finally induced a very high global conversion rate, followed by a relatively low decrease of the methane conversion with time after the initial phase. Thus, to our surprise, the kinetic profile of the methane conversion obtained with a membrane reactor comprising a selective hydrogen-permeable membrane was completely different from that of the same conversion performed under identical conditions but with a standard reactor (comprising no selective hydrogen-permeable membrane), as shown by Curve (1) of Figure 1 (see Example 3 comparative). Indeed, Curve (1) showed a very low initial catalyst activity which exhibited no maximum conversion and remained extremely low during the initial reaction phase (more than 20 times lower than in Curve (2)). Furthermore, in the absence of the selective hydrogen-permeable membrane, the conversion rate increased very slowly with time and the final conversion rate remained very substantially lower than in Curve (2), and the global conversion rate remained at a level much lower than in Curve (2). In the process of the invention, it could be normally envisaged that the use of a selective hydrogen-permeable membrane would reduce the proportion of the hydrogen in the reaction mixture and shift the reaction towards the formation of ethane. Under these conditions, it would have been expected that the kinetic curve of the reaction would have been only shifted uniformly by homothety to some higher conversions with the same kinetic profile and particularly with a very low initial catalyst activity exhibiting no maximum conversion during the initial reaction phase, as shown by the dotted curve in Figure 1. Because of a very different and new kinetic profile, Curve (2) of Figure 1 clearly showed that when using a selective hydrogen-permeable membrane in the process of the present invention, the metal catalyst unexpectedly got activated extremely quickly to its full potential to steady state activity. In other words, the activation time of the metal catalyst is strongly accelerated so as to reach a maximum catalyst activity, or a full potential to steady state catalyst activity in a very short time during the initial reaction phase of the conversion. In addition, it also resulted from the new kinetic profile that the global conversion rate obtained with the membrane reactor was extremely high and much higher than the one expected by the only shifting of a reverse reaction. Furthermore, contrary to the general teaching of International Patent Application WO 02/053520 showing activation or regeneration of similar metal catalysts by use and/or addition of hydrogen into alkane metathesis reactions, it was unexpectedly found in the present invention that a substantial reduction of the proportion of the hydrogen in the reaction mixture obtained by using a selective hydrogen-permeable membrane led to extremely quick activation of the catalyst to its steady state activity.

One of the main advantages of the present process is to provide a methane conversion rate which very quickly reaches a maximum and an extremely high level in the initial reaction phase of the conversion, so that the global methane conversion rate is very high, while maintaining a very high level of the selectivity for ethane.

The following examples illustrate the present invention.

### Example 1: preparation of a metal catalyst (C1) comprising tungsten grafted onto an alumina support.

In a first stage, 2g of a γ-alumina (gamma-alumina) containing 90 wt % of alumina and 9 wt % of water, and sold by Johnson Matthey (Great Britain) were subjected to a calcination treatment under a dry air current at 500°C for 15 hours, then to a dehydroxylation treatment under an absolute pressure of 10⁻² Pa at 500°C for 15 hours. At the end of this time, the alumina thus treated was cooled under an argon atmosphere to 25°C.

In a second stage, 2 g of the alumina prepared beforehand were isolated and introduced under an argon atmosphere into a reactor fitted with a magnetic agitator at 25°C. Then, 156 mg of a precursor (Pr), namely tungsten tris(neopentyl)neopentylidyne of general formula (9):

W[-CH₂-C(CH₃)₃]₃[≡C-C(CH₃)₃] (9)

were introduced into the reactor.

The reactor was heated to 66°C and the mixture thus obtained was agitated in the dry state for 4 hours. At the end of this time, the reactor was cooled to 25°C under an argon atmosphere, and the solid mixture was then washed with n-pentane at 25°C. The solid compound thus washed was vacuum dried, then isolated under an argon atmosphere, so as to obtain an organometallic tungsten compound grafted onto alumina, containing 2.0 wt % of tungsten.

In a third stage, 40 mg of the organometallic tungsten compound grated onto alumina prepared beforehand were isolated and subjected in a reactor to a hydrogenation treatment by contacting with hydrogen under an absolute hydrogen pressure of 73 kPa at 150°C for 15 hours. At the end of this time, the reactor was cooled to 25°C, and a metal catalyst (C1) based on an organometallic tungsten hydride grafted onto alumina and containing 2.0 wt % of tungsten was obtained and isolated under an argon atmosphere.

### Example 2: preparation of a metal catalyst (C2) comprising tantalum grafted onto a silica support.

In a first stage, 1 g of a silica sold by Degussa (Germany) under the trade name "Aerosil 200"® was subjected to a dehydroxylation treatment under an absolute pressure of 10⁻² Pa at 700°C for 15 hours. At the end of this time, the silica thus treated was cooled to 25°C under an argon atmosphere.

In a second stage, 1 g of the silica prepared beforehand was isolated and introduced into a reactor at 25°C under an argon atmosphere. 127 mg of a precursor (Pr'), namely tantalum tris(neopentyl)neopentylidene of general formula (10):

Ta [-CH2-C(CH3)3]3[=CH-C(CH3)3] (10)

in solution in 10 ml of pentane were introduced into the reactor.

The reactor was then stirred at 25°C for 2 hours, so as to produce an organometallic tantalum compound grafted onto silica. At the end of this time, the excess of unreacted precursor (Pr') was eliminated by washings with pentane. The organometallic tantalum compound thus grafted onto silica was isolated under an argon atmosphere and contained 2.0 wt % of tantalum.

In a third stage, the organometallic tantalum compound grafted onto silica prepared beforehand was subjected to a hydrogenolysis treatment by contacting with hydrogen under an absolute hydrogen pressure of 73 kPa at 150°C for 15 hours. At the end of this time, the reactor was cooled to 25°C, and a metal catalyst (C2) based on an organometallic tantalum hydride grafted onto silica and containing 2.0 wt % of tantalum was obtained and isolated under an argon atmosphere.

### Example 3 (comparative): conversion of methane into ethane in a standard reactor.

As a comparative example, conversion of methane into ethane by a non-oxidative methane coupling reaction was carried out in a standard gas phase reactor comprising no selective hydrogen-permeable membrane, in the following way.

Methane at a continuous flow rate, R', of 3 ml/min (i.e. 1.33 x 10⁻⁴ mol of methane per minute) and under a total absolute pressure, P', of 5 MPa was continuously introduced into and passed through a (standard) gas phase dynamic reactor previously heated to 350°C and containing 0.5 g of the metal catalyst (C1) prepared in Example 1, mixed and diluted with 8 g of the γ-alumina as prepared and isolated at the end of the first stage of Example 1.

Contacting the methane with the metal catalyst (C1) continuously during a time of 8,000 minutes under these conditions resulted in the production of ethane with a selectivity by weight for ethane with respect to carbon-containing products produced which was greater than 99.9 %. In particular, no detectable traces of alkenes or alkynes, such as ethylene or acetylene, or aromatic compounds, such as benzene, or carbon monoxide, or carbon dioxide were found.

Methane was continuously converted during 8,000 minutes according to Curve (1), as shown in Figure 1, expressing the percentage of methane conversion (C %) versus time (T) in minutes. Curve (1) was characterised by an initial conversion percentage (₍ᵢ₎C %) of 0.02 % (after 550 minutes) and a final conversion percentage (_{(f)}C %) of 0.12 % (after 8,000 minutes). Curve (1) was characterised by a kinetic profile comprising a very low initial catalyst activity and then a catalyst activity progressing relatively slowly with time and finishing at a relatively low level after 8,000 minutes, so that the global methane conversion remained at a low level.

### Example 4: conversion of methane into ethane in a membrane reactor.

As an example of the invention, conversion of methane into ethane by a non-oxidative methane coupling reaction was carried out exactly as in Example 3 (comparative), apart from the fact that a gas phase dynamic sweep-flow membrane reactor comprising a selective hydrogen-permeable membrane was used instead of the standard gas phase dynamic reactor.

More particularly, the selective hydrogen-permeable membrane is a dense metallic membrane supported on a porous metallic support, especially a palladium/silver (75/25 b.w.) alloy layer coated by a palladium film and supported on a chromium-nickel alloy support, sold by REB Research & Consulting (USA). The membrane arranged in the membrane reactor comprised a reaction side and a permeate side. The reaction side of the membrane was facing the metal catalyst (C1) and the reaction mixture produced in the membrane reactor, i.e. the interior of the membrane reactor wherein methane was continuously introduced and contacted with the metal catalyst (C1) under an absolute total pressure (P'). The permeate side of the membrane was facing the exterior of the membrane reactor and was submitted to an external absolute pressure, P", so that it resulted a pressure difference (ΔP = P' - P") through the membrane. The permeate side of the membrane was also subjected to a sweep gas flow consisting of argon under the external absolute pressure (P") and flowing at a continuous flow rate (R"), so as to form a permeate gas mixture comprising the argon and the hydrogen having permeated the membrane.

Thus, methane at a continuous flow rate (R') of 3 ml/min (i.e. 1.33 x 10-4 mol of methane per minute) and under a total absolute pressure (P') of 5 MPa was continuously introduced into and passed through the membrane reactor previously heated to 350°C and containing 0.5 g of the metal catalyst (C1) prepared in Example 1, mixed and diluted with 8 g of y-alumina as prepared and isolated at the end of the first stage of Example 1. The external absolute pressure (P") was continuously maintained at 4.5 MPa, so that the pressure difference (ΔP) through the membrane was equal to 0.5 MPa, and the argon used as a sweep gas was continuously flowing at a continuous flow rate (R") of 3 ml/min under the pressure (P") and at a temperature of 350°C.

Contacting the methane with the metal catalyst (C1) continuously during a time of 8,000 minutes under these conditions resulted in the production of ethane with a selectivity by weight for ethane with respect to carbon-containing products produced which was very high and greater than 99.9 %. In particular, no detectable traces of alkenes or alkynes, such as ethylene or acetylene, or aromatic compounds, such as benzene, or carbon monoxide, or carbon dioxide were found.

Methane was continuously converted during a time of 8,000 minutes according to Curve (2), as shown in Figure 1, expressing the percentage of methane conversion (C %) versus time (T) in minutes. Curve (2) was characterised by an initial conversion percentage (₍ᵢ₎C %) of 0.45 % (after 500 minutes) and a final conversion percentage (_{(f)}C %) of 0.25 % (after 8,000 minutes). Curve (2) showed a kinetic profile which was completely different from that in Curve (1) (Example 3 (comparative)), and exhibited an extremely high initial catalyst activity (about 23 times higher than in Curve (1)), and a final catalyst activity maintained at a very high level after 8,000 minutes (about 2 times higher than in Curve (1)). The new kinetic profile clearly suggested that the use of a membrane reactor was unexpectedly able to accelerate the catalyst activation time from about 3,000 minutes in a standard case to less than 500 minutes, and more particularly to provide an extremely high maximum catalyst activity during the initial reaction phase and finally a global methane conversion maintained at a very high level, compared to those obtained in a standard reactor as shown by Example 3 (comparative).

### Example 5 (comparative): conversion of methane into ethane in a membrane reactor.

As a comparative example, conversion of methane into ethane was attempted to be carried out exactly as in Example 4, apart from the fact that no metal catalyst (C1) was used.

It was observed no conversion of methane took place. It resulted that the selective hydrogen-permeable membrane used in the membrane reactor did not induce any specific chemical or catalytic effect on methane.

### Example 6: conversion of methane into ethane in a membrane reactor.

As an example of the invention, conversion of methane into ethane was carried out exactly as in Example 4, apart from the fact that some operating conditions were varied with time, so as that three different periods, Periods (1), (2) and (3), were identified in the methane conversion, such as respectively shown by Curves (1), (2) and (3) in Figure 2.

In Period (1) (from 0 to 2,700 minutes), methane at a continuous flow rate (R') of 3 ml/min (i.e. 1.33 x 10⁻⁴ mol of methane per minute) and under a total absolute pressure (P') of 5 MPa was continuously introduced into and passed through the membrane reactor previously heated to 350°C and containing 1.5 g of the metal catalyst (C1) prepared in Example 1, mixed and diluted with 7 g of γ-alumina as prepared and isolated at the end of the first stage of Example 1. The external absolute pressure (P") was maintained at 4.5 MPa, so that the pressure difference (ΔP) through the membrane was equal to 0.5 MPa, and the argon used as a sweep gas was continuously flowing at a continuous flow rate (R") of 3 ml/min under the pressure (P") and at a temperature of 350°C.

Contacting the methane with the metal catalyst (C1) continuously during Period (1) under these conditions resulted in the production of ethane with a selectivity by weight for ethane with respect to carbon-containing products produced which was very high, greater than 99.9 % and similar to that obtained in Example 4. No detectable traces of alkenes or alkynes, such as ethylene or acetylene, or aromatic compounds, such as benzene, or carbon monoxide, or carbon dioxide were found. Methane was converted during Period (1) according to Curve (1) in Figure 2, expressing the percentage of methane conversion (C %) versus time (T) in minutes. Curve (1) was characterised by an initial conversion percentage (₍ᵢ₎C %) of about 0.39 % (after 550 minutes) and a conversion percentage of about 0.38 % after 2,700 minutes (the end of Period (1)) which were extremely high and very similar to those obtained in Example 4.

In Period (2) (from 2,700 to 4,100 minutes), all the operating conditions applied in Period (1) were maintained, apart from the fact that the external absolute pressure (P") was decreased to 4.0 MPa, so that the pressure difference (ΔP) through the membrane was increased to 1.0 MPa. It was observed that ethane was continuously produced during Period (2) with a very high selectivity by weight for ethane similar to that obtained in Example 4, and no detectable traces of the above-mentioned poisons were found. Methane was converted during Period (2) according to Curve (2) in Figure 2, expressing the percentage of methane conversion (C %) versus time (T) in minutes. Curve (2) was characterised by an initial conversion percentage (₍ᵢ₎C %) of about 0.40 % and a conversion percentage of about 0.36 % at the end of Period (2) which were extremely high and similar to those obtained in Example 4.

In Period (3) (from 4,100 to 5,900 minutes), all the operating conditions applied in Period (1) were maintained, apart from the fact that methane was continuously introduced into the membrane reactor with a continuous flow rate (R') increased to 6 ml/min (i.e. 2.66 x 10⁻⁴ mol of methane per minute). It was observed that ethane was continuously produced during Period (3) with a very high selectivity by weight for ethane similar to that obtained in Example 4, and no detectable traces of the above-mentioned poisons were found. Methane was converted during Period (3) according to Curve (3) in Figure 2, expressing the percentage of methane conversion (C %) versus time (T) in minutes. Curve (3) was characterised by an initial conversion percentage (₍ᵢ₎C %) of about 0.35 % and a conversion percentage of about 0.34 % at the end of Period (3) which were extremely high and similar to those obtained in Example 4.

All the kinetic profiles thus obtained in Example 6 again clearly indicated that when the methane non-oxidative coupling reaction was performed in a membrane reactor comprising a selective hydrogen-permeable membrane, the catalyst activation time was accelerated very significantly (by more than 20 times) and an extremely high maximum catalyst activity was obtained during the initial reaction phase, and finally a global methane conversion was maintained at a very high level.

### Example 7: conversion of methane into ethane in a membrane reactor.

As an example of the invention, conversion of methane into ethane was carried out exactly as in Example 4, apart from the fact that the metal catalyst (C2) was used instead of the metal catalyst (C1).

Contacting the methane with the metal catalyst (C2) continuously during a time of 8,000 minutes under these conditions resulted in the production of ethane with a selectivity by weight for ethane with respect to carbon-containing products produced which was very high and similar to that in Example 4.

Methane was thus continuously converted during 8,000 minutes, with an initial conversion percentage (₍ᵢ₎C %) (after 550 minutes) and a final conversion percentage (_{(f)}C %) (after 8,000 minutes) which were extremely high and very similar to those in Example 4. Thus, the kinetic profile in Example 7 clearly suggested that in a membrane reactor comprising a selective hydrogen-permeable membrane, the catalyst was able to reach a very high maximum activity very quickly during the initial reaction phase of the conversion, and finally a global methane conversion that was maintained at a very high level.

## Claims

1. Process for producing ethane comprising contacting methane with a metal catalyst selected from metal hydrides, organometallic compounds, organometallic hydrides and metal clusters, in a reaction zone and forming a reaction mixture comprising ethane and hydrogen, **characterised in that** the reaction zone comprises a membrane reactor equipped with a selective hydrogen-permeable membrane.

2. Process according to Claim 1, **characterised in that** the methane is used alone or in the form of a mixture of methane with 1 % by mole or less of one or more other alkanes preferably selected from the (C₂₊) alkanes having 2 carbon atoms and more.

3. Process according to Claim 1, **characterised in that** the methane is used in the form of a mixture of methane with one or more other alkanes preferably selected from the (C₂₊) alkanes having 2 carbon atoms and more, the methane being in a molar proportion higher than 50 % and lower than 99 %, preferably higher than 60 % and lower than 99 %, particularly higher than 75 % and lower than 99 %.

4. Process according to Claim 1, **characterised in that** the methane is used in the form of natural gas.

5. Process according to any one of Claims 1 to 4, **characterised in that** the metal catalyst is selected from supported metal clusters preferably comprising one or more metals of Groups 4, 5 and/or 6 of the Periodic Table of the Elements, particularly tantalum.

6. Process according to any one of Claims 1 to 4, **characterised in that** the metal catalyst is selected from metal hydrides, organometallic compounds and organometallic hydrides, supported on and preferably grafted onto a solid support.

7. Process according to Claim 6, **characterised in that** the metal catalyst comprises one or more metals chosen from lanthanides, actinides and metals of Groups 2 to 12, preferably from transition metals of Groups 3 to 12, particularly of Groups 3 to 10 of the Periodic Table of the Elements.

8. Process according to Claim 6 or 7, **characterised in that** the metal catalyst chosen from organometallic compounds and organometallic hydrides comprises one or more hydrocarbon radicals (R), either saturated or unsaturated, linear or branched, preferably having from 1 to 20, particularly from 1 to 14 carbon atoms.

9. Process according to any one of Claims 6 to 8, **characterised in that** the solid support is chosen from metal oxides, metal sulphides, metal carbides, metal nitrides, metal salts, refractory oxides, refractory sulphides, refractory carbides, refractory nitrides and refractory salts, and from carbon, mesoporous materials, organic or inorganic hybrid materials, metals and molecular sieves.

10. Process according to any one of Claims 1 to 9, **characterised in that** the contacting is performed at a temperature chosen in a range of from 150 to 700°C, preferably from 200 to 650°C, particularly from 250 to 600°C, especially from 300 to 600°C.

11. Process according to any one of Claims 1 to 10, **characterised in that** the contacting is performed under an absolute total pressure (P') chosen in a range of from 0.1 to 50 MPa, preferably from 0.1 to 30 MPa, particularly from 0.1 to 20 MPa.

12. Process according to any one of Claims 1 to 11, **characterised in that** the contacting comprises adding the methane to the metal catalyst, or adding the metal catalyst to the methane, or adding the methane and the metal catalyst simultaneously into the reaction zone, preferably continuously.

13. Process according to any one of Claims 1 to 12, **characterised in that** the selective hydrogen-permeable membrane is selected from microporous inorganic membranes and dense inorganic membranes, preferably supported on a porous inorganic support particularly selected from porous metallic supports, porous steel supports, porous ceramic supports, porous glass supports and porous refractory oxide supports.

14. Process according to Claim 13, **characterised in that** the microporous inorganic membrane is selected from microporous silica membranes, microporous alumina membranes, microporous zirconia membranes, microporous aluminium-doped silica membranes, microporous binary alumina-silica membranes, microporous binary zirconia-silica and microporous binary zirconia-alumina membranes.

15. Process according to Claim 13, **characterised in that** the microporous inorganic membrane is selected from zeolite membranes, preferably from polycrystalline zeolite membranes.

16. Process according to Claim 13, **characterised in that** the dense inorganic membrane is selected from dense metallic membranes, dense ceramic membranes and dense cermet (ceramic-metal) membranes, preferably supported on a porous inorganic supports particularly selected from porous metallic supports, porous steel supports, porous ceramic supports, porous glass supports and porous refractory oxide supports.

17. Process according to Claim 16, **characterised in that** the dense metallic membrane comprises one or more metals selected from Groups 4 to 11, preferably from Groups 4 to 6 and Groups 8 to 11, particularly from Groups 8 to 11, more particularly from Group 10 of the Periodic Table of the Elements.

18. Process according to Claim 17, **characterised in that** the dense metallic membrane comprises one or more metals selected from nickel, palladium and platinum, preferably palladium or palladium alloy particularly comprising palladium and one or more other metals selected from cerium, yttrium, ruthenium, osmium, nickel, platinum, copper, silver and gold, especially silver.

19. Process according to Claim 18, **characterised in that** the dense metallic membrane is a palladium and/or palladium alloy membrane, preferably a palladium and/or palladium-silver alloy membrane, particularly supported on a porous metallic or ceramic support.

20. Process according to Claim 16, **characterised in that** the dense ceramic membrane is selected from proton-conducting ceramic membranes, preferably from perovskite type proton-conducting ceramic membranes.

21. Process according to Claim 20, **characterised in that** the proton-conducting ceramic membrane is based on the general formula:
A B₍₁₋ₓ₎B'ₓ O₃
wherein A represents a calcium, strontium or barium atom, preferably a strontium or barium atom, B represents an atom of cerium, B' represents a titanium, vanadium, chromium, manganese, iron, copper, yttrium, praseodymium, terbium, thulium or ytterbium atom, preferably a thulium atom, and x is a number of from 0 to 0.2, preferably from 0 to 0.1.

22. Process according to any one of Claims 1 to 21, **characterised in that** the membrane reactor is equipped with the selective hydrogen-permeable membrane comprising a reaction side facing the metal catalyst and the reaction mixture present in the interior of the membrane reactor under an absolute total pressure (P'), and a permeate side facing the exterior of the membrane reactor submitted to an external absolute pressure (P") lower than the pressure (P'), so that it results a pressure difference (ΔP = P' - P") through the membrane preferably selected in a range of from 0.05 to 5 MPa, particularly from 0.1 to 2 MPa, more particularly from 0.2 to 1 MPa.

23. Process according to Claim 22, **characterised in that** the permeate side of the membrane is subjected to a sweep gas flow preferably selected from inert gases, particularly from nitrogen, argon and helium, in particular so as to entrain the hydrogen externally to the membrane and to form a permeate gas mixture comprising the hydrogen and the sweep gas.

24. Process according to Claim 23, **characterised in that** the membrane reactor is selected from sweep-flow membrane reactors.

25. Use of a membrane reactor equipped with a selective hydrogen-permeable membrane for activating a metal catalyst to reach its maximum activity during the initial reaction phase in a process for converting methane into ethane comprising contacting methane with said metal catalyst selected from metal hydrides, organometallic compounds, organometallic hydrides and metal clusters, and forming a reaction mixture comprising ethane and hydrogen.
